# EUROPEAN PATENT APPLICATION

(11) **EP 4 285 946 A1**
(43) Date of publication of application: **06.12.2023**
(21) Application number: 22382540.7
(22) Date of filing: 03.06.2022
(51) Int. Cl.: A61L 17/04, A61L 17/08, A61L 17/14

(54) **SURGICAL THREAD, SURGICAL SUTURE AND SURGICAL KIT**

(71) Applicant: B. Braun Surgical, S. A., 08191 Rubi (ES)
(72) Inventor: Turón Dols, Pau, 08191 Rubí (ES); Funk, Lutz, 08191 Rubí (ES); Weis, Christine, 08191 Rubí (ES); Ibáñez, Núria, 08191 Rubí (ES); Lorenzo, Sandra, 08191 Rubí (ES)
(74) Representative: Patentanwälte Ruff, Wilhelm, Beier, Dauster & Partner mbB

(57) **Abstract**

The invention relates to a surgical thread comprising
- a thread body comprising or consisting of a self-sticking material being capable of sticking to a biological tissue upon contact with the biological tissue and/or an activator or
- a thread body and a coating, wherein the coating at least partially surrounds the thread body, wherein the coating comprises or consists of a self-sticking material being capable of sticking to a biological tissue upon contact with the biological tissue and/or an activator.

The invention further relates to a surgical device, a surgical kit and a method for preparing the surgical thread.

## Description

### FIELD OF THE INVENTION

The present invention relates to a surgical thread, a surgical suture and a surgical kit.

### BACKGROUND OF THE INVENTION

Surgical sutures are a commonly used standard medical devices for closing or binding together wounds in human or animal tissues, such as skin, muscles, tendons, internal organs, nerves, blood vessels, and the like. Typically, the surgeon applies a surgical needle with an attached conventional suture in order to pierce the tissue alternately on opposing faces of the wound and thus sew the wound closed. After removal of the surgical needle, the ends of the suture threads are tied. In that regard, carefulness has to be applied in order to close the wounds with an optimal force at the wound margins. If the wound margins are sutured too loosely and too irregularly, for example, there is a risk of increased scarring or dehiscence. By contrast, too strongly sutured wound margins may result in ischemia and even in necrosis of the affected tissue.

Further, several knots are typically required to achieve a secure wound closure. However, this entails the introduction of a large amount of suture material into the wound zone which may cause undesired foreign-body reactions.

Furthermore, there exists a risk of sliding in the knot/knots which may result in an undesired premature loosening of the wound closure.

Recently, knotless sutures have been developed. These sutures are based on the concept of barbs protruding from the surface of the suture body. The barbs are formed on the suture body in such a way that the suture can be pulled through the tissue along the direction of the barbs without any great resistance and without tissue trauma. When a pull is exerted in the opposite direction, however, the bards stand upright and anchor themselves, and therefore also the suture, in the surrounding tissue area. This ensures that the suture cannot be pulled back through the incision channel. Thus, in this case, wound closure may be accomplished not by knotting the suture but by physical retention in the tissue due to the barbs of the suture. Barbed sutures are disclosed, for example, in US 3,123,077, EP 1 559 266 B1, EP 1 560 683 B1 and EP 1 556 946 B1.

It is however principally disadvantageous that the physical retention of barbed sutures may result in undesired tissue trauma and the risk of achieving a low retention strength. In addition, the formation of barbs on a suture body results in an at least portion-like reduction of the suture diameter which may impair the mechanical stability of the suture.

### OBJECT AND SOLUTION

In view of the foregoing, an object underlying the present invention is therefore to make available a surgical thread, which circumvents disadvantages, in particular as described above, in the context of conventional sutures. Further objects of the present invention refer to the provision of a surgical suture and a surgical kit.

These objects are accomplished by a surgical thread according to independent claim 1, a surgical device according to claim 14, a surgical kit according to claim 15 and a method for preparing a surgical thread as disclosed in the description. Preferred embodiments of the invention are defined in the dependent claims and the description. The subject-matter and wording, respectively, of all claims is hereby incorporated into the description by explicit reference.

According to a first aspect, the present invention refers to a surgical thread comprising
- a thread body comprising or consisting of a self-sticking (self-adhesive) material being capable of sticking to or gluing with a biological, in particular human or animal, tissue upon contact with the biological, in particular human or animal, tissue and/or upon contact with an activator
or
- a thread body and a coating, wherein the coating at least partially, in particular only partially or continuously, i.e. completely, surrounds or covers the thread body, wherein the coating comprises or consists of a self-sticking (self-adhesive) material being capable of sticking to or gluing with a biological, in particular human or animal, tissue upon contact with the biological, in particular human or animal, tissue and/or upon contact with an activator.

Accordingly, the surgical thread according to the present invention may also be termed as a self-sticking or self-adhesive surgical thread.

The term "self-sticking surgical thread" or "self-adhesive surgical thread" as used according to the present invention refers to a surgical thread which is capable of sticking to or gluing with a biological, in particular human or animal, tissue upon contact with the biological, in particular human or animal, tissue and/or an activator.

As already mentioned above, the term "self-sticking material" or self-adhesive material" as used according to the present invention refers to a material which is capable of sticking to or gluing with a biological, in particular human or animal, tissue upon contact with the biological, in particular human or animal, tissue and/or upon contact with an activator.

The biological tissue may be, for example, selected from the group consisting of skin, muscles, tendons, internal organs, nerves and blood vessels.

The term "animal tissue" as used according to the present invention refers to a tissue from animal, except human, origin.

Advantageously, the surgical thread according to the present invention facilitates wound closure and/or wound healing without the necessity of knotting the suture and concurrently avoids disadvantages known in respect of physical retention of barbed sutures such as undesired tissue trauma. This is particularly useful in minimally invasive approaches, in particular minimally invasive laparoscopic approaches, where the knotting process is much more complex due to the reduced working area and the use of instruments to sew.

Preferably, the self-sticking material is capable of sticking to or gluing with the biological tissue by means of a chemical bonding, in particular by means of a covalent bonding and/or non-covalent bonding. The non-covalent bonding may be in particular selected from the group consisting of ionic interactions, coordinative bonding, van der Waals forces, hydrogen bonding and combinations of at least two of the afore-said types of non-covalent bonding.

The thread body of the surgical thread may be in the form of a monofilament or fiber, pseudo monofilament or multifilament, in particular textured, intertwined, twisted or braided multifilament. In particular, the surgical thread body may be in the form of a barbed monofilament or barbed fiber, barbed pseudo monofilament or barbed multifilament.

Further, the thread body may have a cornerless, in particular circular, oval or ellipsoid, cross-section or a polygonal, in particular triangular, square, rectangular, pentagonal, hexagonal, heptagonal, octagonal, nonagonal, decagonal or star-like, cross-section.

Further, the thread body may in particular have a diameter of 0.01 mm to 1.1 mm, in particular 0.01 mm to 0.019 mm or 0.02 mm to 0.029 mm or 0.03 mm to 0.039 mm or 0.04 mm to 0.049 mm or 0.05 mm to 0.059 mm or 0.07 mm to 0.079 mm or 0.1 mm to 0.149 mm or 0.15 mm to 0.199 mm or 0.2 mm to 0.249 mm or 0.25 mm to 0.299 mm or 0.3 mm to 0.349 mm or 0.35 mm to 0.399 mm or 0.4 mm to 0.499 mm or 0.5 mm to 0.599 mm or 0.6 mm to 0.699 mm or 0.7 mm to 0.799 mm or 0.8 mm to 0.899 mm or 0.9 mm to 0.999 mm or 1 mm to 1.099 mm.

Further, the thread body of the surgical thread may comprise or consist of a non-absorbable or absorbable material, in particular polymer, in particular synthetic polymer, i.e. a polymer being produced by chemical synthesis, biotechnology or genetic engineering, or a biopolymer, i.e. a naturally occurring polymer or related polymer thereof. More specifically, the thread body may comprise or consist of a polymer, in particular synthetic polymer, which is preferably selected from the group consisting of polyolefins, polyvinyl alcohols, polyamides, polyimides, polyesters, polyurethanes, in particular thermoplastic polyurethanes, polyhydroxyalkanoates, copolymers thereof, and mixtures of at least two of the afore-said polymers, in particular synthetic polymers.

Further, the thread body may comprise or consist of a polymer, in particular biopolymer or related polymer thereof, in particular selected from the group consisting of proteins, structural proteins, extracellular proteins, fibrous proteins, polysaccharides, oxidized or non-oxidized polysaccharides, amino group bearing polysaccharides, aldehyde group bearing polysaccharides, mucopolysaccharides, salts thereof, stereoisomers thereof, copolymers of at least two of the afore-said polymers and mixtures of at least two of the afore-said polymers, in particular biopolymers and related polymers thereof, respectively.

More specifically, the thread body may comprise or consist of a polymer which is selected from the group consisting of polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyamide 6, polyamide 6-6, polyamide 6-12, polyamide 12, rayon, silk, in particular spider silk, polytetrafluorethylene, polyvinylidene dichloride, polyvinylidene difluoride, polytetrafluorpropylene, polyhexafluorpropylene, polyvinyl alcohol, polyglycolide or polyglycolic acid, polylactide or polylactic acid, polydioxanone, polyhydroxybutyrate or polyhydroxybutyric acid, poly-3-hydroxybutyrate or poly-3-hydroxybutyric acid, poly-4-hydroxybutyrate or poly-4-hydroxybutyric acid, polytrimethylene carbonate, poly-ε-caprolactone, collagen, gelatine, elastin, reticulin, fibronectin, laminin, fibrin, albumin, starch, amylose, amylopectin, dextran, dextrin, cellulose, cellulose derivatives such as methylcellulose, hydroxymethylcellulose, hydroxyethylcellulose, hydroxypropylcellulose, carboxymethylcellulose, chitin, chitosan, hyaluronic acid, dextran sulfate, heparin, heparan sulfate, chondroitin sulfate, dermatan sulfate, salts thereof, stereoisomers thereof, copolymers of at least two of the afore-said polymers and mixtures of at least two of the afore-said polymers.

Further, the coating may be a textile coating or a non-textile coating. Preferably, the coating according to the present invention is in the form of a non-textile coating.

Further, the coating may have a proportion of > 0.001 percent by weight to 50 percent by weight, in particular 0.01 percent by weight to 25 percent by weight, preferably 0.1 percent by weight to 10 percent by weight, based on the total weight of the surgical thread.

Further, the coating may have a thickness, in particular a constant or non-constant thickness, of 10 nm to 1 mm, in particular 100 nm to 0.1 mm, preferably 1 µm to 100 µm, in particular all over the length of the thread body.

Further, the coating may be covalently and/or non-covalently bonded or fixed (i.e. attached) to the thread body. In particular, the coating may be bonded or fixed to the thread body by means of covalent bonding and/or ionic interactions and/or coordinative bonding and/or van der Waals forces and/or hydrogen bonding.

In an embodiment of the invention, the activator is a biological activator.

The term "biological activator" as used according to the present invention refers to a naturally occurring substance/compound or liquid.

For example, the activator, in particular biological activator, may be a protein, a cell membrane protein, a peptide, a growth factor, a cytokine, a sugar, a lipid, a phospholipid, a nucleoside, a nucleic acid or mixtures of at least two of the afore-said activators, in particular biological activators.

In a further embodiment of the invention, the activator is a chemical activator.

The term "chemical activator" as used according to the present invention refers to a chemical substance (chemical compound), solid, liquid or gas. Preferably, the chemical activator is a chemical substance or chemical compound, more preferably a functionalized chemical substance or functionalized chemical compound. The term "functionalized chemical substance" or "functionalized chemical compound" as used according to the present invention refers to a substance or compound respectively, bearing or carrying at least one functional group. As regards useful functional groups, reference is made to the functional groups disclosed in the following description in respect of derivatives/modifications of proteins and polysaccharides as examples for possible self-sticking materials or possible parts or components of a self-sticking material, according to the present invention.

For example, the activator, in particular chemical activator, may be a polymerization initiator.

More specifically, the activator, in particular chemical activator, may be in the form of a free radical polymerization activator. The free radical polymerization activator may be in the form of a free radical substance or substance leading to or being convertible into a free radical molecule. The free radical substance may be in particular selected from the group consisting of superoxide (O⁻²), oxygen radical (O^{··2}), hydroxyl (OH^{·}), alkoxyradical (RO^{·}), peroxyl radical (ROO^{·}), nitric oxide (nitrogen monoxide) (NO^{·}) and nitrogen dioxide (NO^{·2}). The high reactivity of these radicals is due to the presence of one unpaired electron which tends to donate it or to obtain another electron to attain stability. The substance leading to or being convertible into a free radical molecule may be in particular selected from the group consisting of hydrogen peroxide (H₂O₂), hypochlorous acid (HOCI), hypobromous acid (HOBr), ozone (O₃), singlet oxygen (¹O₂), nitrous acid (HNO₂), nitrosyl cation (NO⁺), nitroxyl anion (NO⁻), dinitrogen trioxide (N₂O₃), dinitrogen tetraoxide (N₂O₄), nitronium (nitryl) cation (NO₂⁺), organic peroxides (ROOH), aldehydes (HCOR) and peroxynitrite (ONOOH).

Alternatively or in combination, the activator, in particular chemical activator, may be a chemical liquid, preferably an aqueous liquid. For example, the activator, in particular chemical activator, may be an aqueous solution, in particular containing biocompatible additives such as biocompatible salts and/or therapeutic agents such as wound-healing, in particular antiinflammatory, agents and/or analgesics.

Preferably, the activator, in particular chemical or biological activator, is water or a body fluid, i.e. a liquid within a human or animal body. In particular, the body fluid may be an intracellular fluid and/or an extracellular fluid, particularly an intravascular fluid such as blood, an interstitial fluid, a lymphatic fluid or transcellular fluid.

More preferably, the activator, in particular chemical or biological activator, is water or a body fluid, in particular tissue fluid and/or blood.

In a further embodiment of the invention, the activator is a physical activator, in particular light, preferably UV light and/or visible light and/or near infrared (NIR) light.

The term "UV light" as used according to the present invention refers to light having a wavelength from 100 nm to 380 nm.

The term "visible light" as used according to the present invention refers to light having a wavelength from > 380 nm to 750 nm, preferably 400 nm to 750 nm.

The term "near infrared (NIR) light" as used according to the present invention refers to light having a wavelength from > 750 nm to 4000 nm.

Preferably, the term "light" as used according to the present invention refers to light having a wavelength from 100 nm to 4000 nm, in particular 240 nm to 650 nm, preferably 240 nm to 260 nm.

Further, the self-sticking material may be preferably a glue, in particular a medical glue, preferably surgical glue, i.e. a glue being applicable in the field of medicine, preferably surgery.

In a further embodiment of the invention, the self-sticking material comprises or consists of polymerizable monomers. In other words, the self-sticking material may be preferably in the form of a polymerizable material.

In a further embodiment of the invention, the polymerizable monomers are selected from the group consisting of alkyl 2-cyanoacrylate monomers, alkoxyalkyl 2-cyanoacrylate monomers, multifunctional cyanoacrylate monomers and mixtures of at least two of the afore-said polymerizable monomers.

Preferably, the polymerizable monomers are alky 2-cyanoacrylate monomers, in particular n-alkyl 2-cyanoacrylate monomers.

In principle, the alkyl 2-cyanoacrylate monomers may have an alkyl moiety comprising 1 carbon atom to 20 carbon atoms, in particular 1 carbon atom to 8 carbon atoms, preferably 1 carbon atom to 4 carbon atoms.

In particular, the alkyl 2-cyanoacrylate monomers may be selected from the group consisting of methyl 2-cyanoacrylate monomers, ethyl 2-cyanoacrylate monomers, n-propyl 2-cyanoacrylate monomers, isopropyl 2-cyanoacrylate monomers, n-butyl 2-cyanoacrylate monomers, isobutyl 2-cyanoacrylate monomers such as, for example, 1-butyl 2-cyanoacrylate monomers and/or 2-butyl 2-cyanoacrylate monomers, n-pentyl 2-cyanoacrylate monomers, isopentyl 2-cyanoacrylate monomers such as, for example, 1-pentyl 2-cyanoacrylate monomers, 2-pentyl 2-cyanoacrylate monomers and/or 3-pentyl 2-cyanoacrylate monomers, cyclopentyl 2-cyanoacrylate monomers, n-hexyl 2-cyanoacrylate monomers, isohexyl 2-cyanoacrylate monomers such as, for example, 1-hexyl 2-cyanoacrylate monomers, 2-hexyl 2-cyanoacrylate monomers, 3-hexyl 2-cyanoacrylate monomers and/or 4-hexyl 2-cyanoacrylate monomers, cyclohexyl 2-cyanoacrylate monomers, n-heptyl 2-cyanoacrylate monomers, isoheptyl 2-cyanoacrylate monomers such as, for example, 1-heptyl 2-cyanoacrylate monomers, 2-heptyl 2-cyanoacrylate monomers, 3-heptyl 2-cyanoacrylate monomers and/or 4-heptyl 2-cyanoacrylate monomers, n-octyl 2-cyanoacrylate monomers, isooctyl 2-cyanoacrylate monomers such as, for example, 1-octyl 2-cyanoacrylate monomers, 2-octyl 2-cyanoacrylate monomers, 3-octyl 2-cyanoacrylate monomers and/or 4-octyl 2-cyanoacrylate monomers, n-nonyl 2-cyanoacrylate monomers, isononyl 2-cyanoacrylate monomers, n-decyl 2-cyanoacrylate monomers, isodecyl 2-cyanoacrylate monomers, n-undecyl 2-cyanoacrylate monomers, isoundecyl 2-cyanoacrylate monomers, n-dodecyl 2-cyanoacrylate monomers, isododecyl 2-cyanoacrylate monomers, and mixtures of at least two of the afore-said alkyl 2-cyanoacrylate monomers.

Especially preferably, the polymerizable monomers are n-butyl 2-cyanoacrylate monomers and/or n-octyl 2-cyanoacrylate monomers.

The above-mentioned alkoxyalkyl 2-cyanoacrylate monomers may be selected from the group consisting of methoxyisopropyl 2-cyanoacrylate monomers, ethoxyethyl 2-cyanoacrylate monomers, isopropoxyethyl 2-cyanoacrylate monomers, 2-butoxyethyl 2-cyanoacrylate monomers, 2-methoxyethyl 2-cyanoacrylate monomers, and mixtures of at least two of the afore-said alkoxyalkyl 2-cyanoacrylate monomers.

The above-mentioned multifunctional cyanoacrylate monomers may be selected from the group consisting of bis-cyanoacrylate monomers, tris-cyanoacrylate monomers and mixtures thereof.

In a further embodiment of the invention, the self-sticking material further comprises a viscosity-modifying, in particular viscosity-reducing, agent. Thus, the viscosity of the self-sticking material may be advantageously adjusted in a targeted manner. For example, in case of a viscosity-reducing agent, the formation of droplets may be avoided.

In a further embodiment of the invention, the viscosity-modifying, in particular viscosity-reducing, agent is an acid, in particular a weak acid. Thus, polymerization of the polymerizable monomers, and thus curing of the self-sticking material until use may be advantageously inhibited. The term "weak acid" as used according to the present invention refers to an acid having a pKₐ value of > 8 to 14. Preferably, the acid is selected from the group consisting of carboxylic acids, amino acids, nucleic acids and mixtures of at least two of the afore-said acids.

Further, the viscosity-modifying, in particular viscosity-reducing, agent may have a proportion of 1 ppb (part per billion) to 50 percent by weight, in particular 1 ppm (part per million) to 0.1 percent by weight, preferably 10 ppm to 0.01 percent by weight, based on the total weight of the self-sticking material.

In a further embodiment of the invention, the self-sticking material further comprises a polymerization retarder. Thus, an undesired premature polymerization of the polymerizable monomers, and thus curing of the self-sticking material may be advantageously avoided. In particular, the polymerization retarder is selected from the group consisting of acids, acidic gases, acid anhydrides, polar substances, non-polar substances, ionic substances, non-ionic substances and mixtures of at least two of the afore-said polymerization retarders.

The acids mentioned with respect to the polymerization retarder may be in particular strong acids and/or medium strong acids.

The term "strong acid" as used according to the present invention refers to an acid having a pKₐ value of -10 to 3.75. The term "medium strong acid" as used according to the present invention refers to an acid having a pKₐ value of 4.75, in particular > 4.75, to 8.

For example, the acids mentioned with respect to the polymerization retarder may be selected from the group consisting of phosphoric acid, hydrogen phosphate acid, sulphonic acid, acetic acid, hydrochloric acid, sulfuric acid, sulfonic acids, carboxylic acids and mixtures of at least two of the afore-said acids. The carboxylic acids may be selected from the group consisting of acetic acid, trichloroacetic acid, acrylic acid, methacrylic acid, itaconic acid and mixtures of at least two of the afore-said carboxylic acids.

The acidic gases mentioned with respect to the polymerization retarder may be selected from the group consisting of sulfur dioxide, nitric oxide, carbon dioxide, hydrogen fluoride and mixtures of at least two of the afore-said acidic gases.

The acid anhydrides mentioned with respect to the polymerization retarder may be selected from the group consisting of carboxylic acid anhydrides, phosphoric anhydrides, acidic gases, antimony pentoxide, sulfones, acid chlorides and mixtures of at least two of the afore-said acid anhydrides. The carboxylic acid anhydrides may be selected from the group consisting of itaconic anhydride, maleic anhydride and mixtures of at least two of the afore-said carboxylic acid anhydrides. A suitable phosphoric anhydride may be, for instance, phosphorous pentoxide.

The non-polar substances mentioned with respect to the polymerization retarder may be selected from the group consisting of phenolic antioxidants such as hydroquinone, secondary aromatic amines, benzofuranones, hydroxyl amines and mixtures of at least two of the afore-said non-polar substances.

The ionic substances mentioned with respect to the polymerization retarder may be selected from the group consisting of anionic inhibitors such as Lewis acids, BF₃ complexes, acetic acid, dichloroacetic acid, trifluoroacetic acid, methanesulfonic acid or 1,3-propanesultone, an acid chelate formed of boric acid or a derivative thereof and a selected polyhydroxy compound and mixtures of at least two of the afore-said ionic substances.

Further, the polymerization retarder may be selected from the group consisting of 6-hydroxy-5-[(4-sulfophenyl)azo]-2-naphthalenesulfonic acid, lactone, boron trifluoride, catechol, pyrogallol, p-benzoquinone, 2-hydroxybenzoquinone, p-methoxyphenol, tert-butylcatechol, organic acids, butylated hydroxyanisole, butylated hydroxytoluene, tert-butyl hydroquinone, alkyl sulfate, alkyl sulfite, 3-sulfolene, alkyl sulfone, alkyl sulfoxide, mercaptan, alkyl sulfide, dioxathiolanes and mixtures of at least two of the afore-said polymerization retarders.

Further, the polymerization retarder may have a proportion of > 1 ppb to 20 percent by weight, in particular 1 ppm to 10 percent by weight, preferably 5 ppm to 0.01 percent by weight, based on the total weight of the self-sticking material.

In a further embodiment of the invention, the self-sticking material comprises or consists of a polymer, in particular linear or branched, for example star-like, polymer. Further, the polymer may be in the form of a homopolymer, i.e. a polymer containing only one sort or type of monomer units, or a copolymer, i.e. a polymer containing two or more sorts or types of monomer units. Further, the copolymer may be, for example, in the form of a biopolymer, i.e. a polymer containing (only) two sorts or types of monomer units, a terpolymer, i.e. a polymer containing (only) three sorts or types of monomer units, or a quaterpolymer, i.e. a polymer containing (only) four sorts or types of monomer units. Further, the copolymer may be in the form of a block copolymer, alternating copolymer, periodic copolymer, statistic or random copolymer, stereo block copolymer or gradient copolymer. The term "block copolymer" as used according to the present invention refers to a copolymer comprising two or more homopolymer subunits linked by covalent bonds. Further, the homopolymer subunits may be linked to each other via an intermediate non-repeating subunit, also known as a junction block. The term "alternating copolymer" as used according to the present invention refers to a copolymer having a regular alternating arrangement of monomer units along the polymer chain. The term "periodic copolymer" as used according to the present invention refers to a copolymer having monomer units being arranged in a repeating sequence along the polymer chain. The term "statistical copolymer" as used according to the present invention refers to a copolymer having a sequence of monomer units that follows a statistical rule. If the probability of finding a given type of monomer unit at a particular point in the polymer chain is equal to the mole fraction of that monomer unit in the polymer chain, then the polymer may be referred to as a (truly) "random copolymer". The term "stereo block copolymer" as used according to the present invention refers to a copolymer having blocks of monomer units, wherein the blocks differ only in the tacticity of the monomer units. The term "gradient copolymer" as used according to the present invention refers to a copolymer whose composition of monomer units changes gradually along the polymer chain.

Further, the self-sticking material may comprise or consist of a biopolymer, i.e. a naturally occurring polymer or a polymer having a structure which is identical or similar to a structure of a polymer occurring in nature or which is derived from a polymer occurring in nature. The biopolymer may be isolated from nature or industrially synthesized, for example in a laboratory. Preferably, the biopolymer is a protein and/or polysaccharide, in particular as described in the following.

In a further embodiment of the invention, the self-sticking material comprises or consists of a protein, in particular a cross-linkable protein.

The protein may in particular be selected from the group consisting of gelatin, collagen, reticulin, elastin, fibronectin, laminin, albumin, fibrin, fibrinogen, thrombin, derivatives, i.e. modifications, thereof, and mixtures of at least two of the afore-said proteins.

Preferably, the protein is gelatin and/or collagen. In other words, the self-sticking material preferably comprises or consists of gelatin and/or collagen.

The gelatin may optionally be any type of gelatin which comprises protein that is known in the art, preferably including but not limited to gelatin obtained by partial hydrolysis of animal tissue and/or collagen obtained from animal tissue, including but not limited to animal skin, connective tissue (including but not limited to ligaments, cartilage and the like), antlers or horns and the like, and/or bones, and/or fish scales and/or bones or other components and/or a recombinant gelatin produced using bacterial, yeast, animal, insect, or plant systems or any type of cell culture.

Preferably, the gelatin is from animal origin, in particular mammalian, in particular porcine, bovine or equine, origin. In particular, the gelatin is of pork skins, pork and/or cattle bones, or split cattle hides, or any other pig or bovine source. More preferably, the gelatin is a porcine gelatin, since it has a lower rate of anaphylaxis. Further, the gelatin from animal origin may optionally be of type A (acid treated) or of type B (alkaline treated).

Further, the gelatin from animal origin may be gelatin obtained during a first extraction, which is generally performed at lower temperature, in particular at a temperature of 50 °C to 60 °C. Gelatin produced in this matter may be in the range of 250 bloom to 300 bloom.

Further, the gelatin may have a molecular weight of 90 kDa to 120 kDa, preferably 90 kDa to 110 kDa, in particular 95 kDa to 100 kDa.

Further, the gelatin from animal origin may be gelatin from fish.

Further, the gelatin may be a recombinant gelatin. The recombinant gelatin may be produced by means of a recombinant yeast system (for example Pichia Pastoris) to express specified fragments of type I, alpha human sequence collagen. Alternatively, the recombinant gelatin may comprise or consist of fully synthetic molecules, containing no contaminating components from humans or any animals. By "synthetic" it is meant that the gelatin is preferably produced according to a method selected from chemical synthesis, cell free protein synthesis, cell tissue culture, any type of bacterial, insect or yeast culture, or in plants. The use of synthetic gelatin may eliminate many of the disadvantages associated with tissue-derived materials, including causing undesired immune response. Advantageously, recombinant gelatin can have no allergenicity.

Further, the self-sticking material may comprise or consist of a polysaccharide and/or a polysaccharide derivative, i.e. a modification of a polysaccharide (modified polysaccharide). The polysaccharide and/or polysaccharide derivate may comprise monosaccharide units containing three carbon atoms and/or monosaccharide units containing four carbon atoms and/or monosaccharide units containing five carbon atoms and/or monosaccharide units containing six carbon atoms and/or monosaccharide units containing more than six carbon atoms.

The terms "derivative" and "modification", respectively as used according to the present invention in respect of the terms "protein" and "polysaccharide" may refer to proteins and polysaccharides being additionally functionalized, in particular with functional groups which are typically not present in the respective native protein and native polysaccharide. The functional groups may be in particular selected from the group consisting of hydroxyl groups, carbonyl groups, aldehyde groups, haloformyl groups, carbonate ester groups, carboxylate groups, carboxyl groups, carboalkoxy groups, methoxy groups, hydroperoxy groups, peroxy groups, ether groups, hemiacetal groups, hemiketal groups, acetal groups, ketal groups, orthoester groups, alkylenedioxy groups such as methylenedioxy groups, orthocarbonate ester groups, carboxylic anhydride groups, carboxamide groups, primary amine groups, secondary amine groups, tertiary amine groups, ammonium ion groups, primary ketimine groups, secondary ketimine groups, primary aldimine groups, secondary aldimine groups, imide groups, azide groups, azo groups, cyanate groups, isocyanate groups, nitrate groups, nitrile groups, isonitrile groups, oxime groups, carbamate groups, sulfhydryl groups, sulfide groups, disulfide groups, sulfinyl groups, sulfonyl groups, sulfino groups, sulfo groups, thiocyanate groups, sulfonic acid groups, carbonothioyl groups, carbothioic S-acid groups, carbothioic O-acid groups, thiolester groups, thionoester groups, carbodithioic acid groups and combinations of at least two of the afore-said functional groups.

In a further embodiment of the invention, the self-sticking material further comprises a crosslinking agent, in particular a crosslinking agent that is capable of crosslinking the protein, preferably the cross-linkable protein, more preferably the gelatin and/or collagen, and/or the polysaccharide.

Preferably, the crosslinking agent is a protein, in particular an enzyme, in particular a transglutaminase.

The transglutaminase may be optionally any plant, animal or microbe derived transglutaminase, preferably other than blood derived factor XIII.

Preferably, the transglutaminase is a microbial transglutaminase, in particular derived from *Streptoverticillium mobaraensis.*

Further, the transglutaminase may be present in a composition, in particular having a specific activity level of 40 U/g to 100 U/g, in particular 60 U/g to 100 U/g, preferably 80 U/g to 100 U/g. The composition may further comprise at least one further ingredient, in particular a stabilizer or filler, in particular selected from the group consisting of maltodextrin, hydrolyzed skim milk protein (or any other protein substance), sodium chloride, safflower oil, trisodium phosphate, sodium caseinate, lactose or a mixture of at least two of the afore-said stabilizers or fillers. Further, the composition may have a pH value in a range from 5 to 8.

For example, the transglutaminase or transglutaminase composition may be a commercially available transglutaminase product produced by Ajinomoto Co. (Kawasaki, Japan), in particular Activa TG-TI (in Europe: Activa WM, ingredients: microbial transglutaminase and maltodextrin, activity: 81-135 U/g of Activa), Activa TG-FP (ingredients: hydrolyzed skim milk protein, microbial transglutaminase, activity: 34-65 U/g of Activa TG-FP), Activa TG-GS (ingredients: sodium chloride, gelatin, trisodium phosphate, maltodextrin, microbial transglutaminase, and safflower oil, activity: 47-82 U/g of Activa TG-GS), Activa TG-RM (in Europe: Activa EB, ingredients: sodium caseinate, maltodextrin, and microbial transglutaminase, activity: 34-65 U/g of Activa) and Activa MP (ingredients: microbial transglutaminase, lactose and maltodextrin, activity: 78-126 U/g of Activa).

Other, non-limiting examples of commercially available transglutaminase product include those produced by Yiming Biological Products Co. (Jiangsu, China), in particular selected from the group consisting of TG-B (ingredients: 1% microbial transglutaminase, 99% co-protein, activity: 80-130 U/g of TG-B) and TG-A (ingredients: 0.5% microbial transglutaminase, 99.5% co-protein, activity: 40-65 U/g of TG-A).

Alternatively, the transglutaminase may be optionally extracted from *Streptoverticillium Baldaccii* or a *Streptomyces Hygroscopicus* strain to produce enzyme variants that have been shown to function optimally at lower temperatures, in particular temperatures of 37 °C to 45 °C.

In a further embodiment of the invention, the coating or the self-sticking material is in the form of a hydrogel.

In a further embodiment of the invention, the self-sticking material is in a dry, in particular lyophilized, form.

More specifically, the above-mentioned protein, preferably cross-linkable protein, more preferably gelatin and/or collagen, and the above-mentioned crosslinking agent, in particular enzyme, preferably transglutaminase, may be in a dry, in particular lyophilized, form. Thus, an undesired prematurely crosslinking of the protein, preferably cross-linkable protein, more preferably gelatin and/or collagen, may be advantageously circumvented.

In a further embodiment of the invention, the surgical thread is in the form of a surgical suture, i.e. a surgical device used to hold body tissues together and approximate wound edges after an injury or surgery. Due to the self-sticking material, there are advantageously no surgical knots necessary to secure the surgical suture and wound closure, respectively, thereby avoiding disadvantages of conventional sutures.

A second aspect of the present invention refers to a surgical device, in particular a textile surgical device, comprising at least one surgical thread, in particular only one surgical thread, or a plurality of, i.e. two or more, surgical threads according to the first aspect of the present invention. More specifically, the surgical device may be in a knitted form, woven form or non-woven form.

Preferably, the surgical device is a surgical suture or in the form of a surgical suture.

With respect to further features and advantages of the surgical device, in particular in respect of the surgical thread, reference is made in its entirety to the previous description. The features and advantages described in the previous description, in particular in respect of the surgical thread, do also apply, mutatis mutandis, with respect to the surgical device according to the second aspect of the invention.

According to a third aspect, the present invention refers to a surgical kit.

The surgical kit comprises, typically spatially separated from each other,
- at least one surgical thread, in particular only one surgical thread or a plurality of, i.e. two or more, surgical threads, according to the first aspect of the present invention or a surgical device according to the second aspect of the present invention
and
- at least one further kit component, preferably selected from the group consisting of instructions for use, a polymerization initiator, an UV-Vis (ultraviolet-visible) lamp, near infrared (NIR) lamp, instructions for use and combinations of at least two of the afore-said further kit components.

With respect to further features and advantages of the kit, in particular in terms of the surgical thread and/or the surgical device, reference is made in its entirety to the previous description. The features and advantages described in the previous description, in particular in terms of the surgical thread and/or surgical device, do apply, mutatis mutandis, with respect to the kit according to the third aspect of the invention.

According to a fourth aspect, the invention relates to a method for preparing or manufacturing a surgical thread, in particular according to the first aspect of the invention, or a surgical device, in particular according to the second aspect of the invention.

The method comprises the step of
- coating a thread body with a self-sticking (self-adhesive) material being capable of sticking to or gluing with a biological, in particular human or animal, tissue upon contact with the biological, in particular human or animal, tissue and/or upon contact with an activator.

The afore-mentioned method step may also be termed as "coating step" according to the present invention.

Principally, the thread body may be only partially or completely coated with the self-sticking material.

More specifically, the coating step may be carried out by immersing the thread body into a liquid comprising or consisting of the self-sticking material. In particular, the coating step may be carried out as a dip coating step.

Alternatively or in combination, the coating step may be carried out by brushing the thread body with a liquid comprising or consisting of the self-sticking material.

Alternatively or in combination, the coating step may be carried out by painting the thread body with a liquid comprising or consisting of the self-sticking material.

Alternatively or in combination, the coating step may be carried out by pouring a liquid comprising or consisting of the self-sticking material over the thread body.

Alternatively or in combination, the coating step may be carried out by spraying the thread body with a liquid comprising or consisting of the self-sticking material.

Alternatively or in combination, the coating step may be carried out by extruding a liquefied or molten mass comprising or consisting of the self-sticking material on the thread body.

With respect to further features and advantages of the method, in particular in terms of the thread body and/or the self-sticking material, reference is made in its entirety to the previous description. The features and advantages described in the previous description, in particular in terms of the thread body and/or the self-sticking material, do apply, mutatis mutandis, with respect to the method according to the fourth aspect of the invention.

Further features and advantages of the invention will become clear from the following description of preferred embodiments in form of examples in conjunction with the subject-matter of the dependent claims. The individual features can be realized either singularly or severally in combination in one embodiment of the invention. The preferred embodiments merely serve for illustration and better understanding of the invention and are not to be understood as in any way limiting the invention.

### EXAMPLE SECTION

### 1. Fiber coated with self-adhesive butyl 2-cyanoacrylate glue

A fiber made of polypropylene/polyethylene (95:5) was pulled, by means of a continuous process at a constant speed remaining in contact with the solution for 2 s seconds, through a dip-coating bath containing a solution composed of butyl 2-cyanoacrylate and a polymerization retardant additive (phosphoric acid) and a thickener (slightly acid substance) to increase the viscosity of the mixture in order to create a self-adhesive thin-film coating of the monomer on the fiber (approximately 1 µm thick). A thin layer deposited itself on the fiber while it was pulled up. The excess of glue was removed with a soft porous material in gentle contact with the fiber. The solvent was allowed to evaporate from the liquid, forming the self-adhesive thin layer. The fiber self-adhered to living tissue when humidity coming from the living tissue activated the polymerization reaction.

### 2. Fiber coated with self-adhesive octyl 2-cyanoacrylate glue

A fiber made of polypropylene/polyethylene (95:5) was pulled, by means of a continuous process at a constant speed remaining in contact with the solution for 2 s seconds, through a dip-coating bath containing a solution composed of octyl 2-cyanoacrylate and a polymerization retardant in order to create a self-adhesive thin-film coating of the monomer on the fiber (approximately 1 µm thick). A thin layer deposited itself on the fiber while it was pulled up. The excess of glue was removed with a soft porous material in gentle contact with the fiber. The solvent was allowed to evaporate from the liquid, forming the self-adhesive thin layer. The fiber self-adhered to living tissue when humidity coming from the living tissue activated the polymerization reaction.

## Claims

1. Surgical thread comprising
- a thread body comprising or consisting of a self-sticking material being capable of sticking to a biological tissue upon contact with the biological tissue and/or an activator or
- a thread body and a coating, wherein the coating at least partially surrounds the thread body, wherein the coating comprises or consists of a self-sticking material being capable of sticking to a biological tissue upon contact with the biological tissue and/or an activator.

2. Surgical thread according to claim 1, **characterized in that** the activator is a biological activator, in particular water or a body fluid, preferably tissue fluid and/or blood.

3. Surgical thread according to claim 1, **characterized in that** the activator is a chemical activator, in particular a chemical substance such as a polymerization initiator.

4. Surgical thread according to claim 1, **characterized in that** the activator is a physical activator, in particular light, preferably UV-light.

5. Surgical thread according to any of the preceding claims, **characterized in that** the self-sticking material comprises polymerizable monomers.

6. Surgical thread according to claim 5, **characterized in that** the polymerizable monomers are selected from the group consisting of alkyl 2-cyanoacrylate monomers, alkoxyalkyl 2-cyanoacrylate monomers, multifunctional cyanoacrylate monomers and mixtures of at least two of the afore-said polymerizable monomers.

7. Surgical thread according to any of the preceding claims, **characterized in that** the self-sticking material further comprises a viscosity-modifying, in particular viscosity-reducing, agent.

8. Surgical thread according to claim 7, **characterized in that** the viscosity-modifying agent is an acid, in particular selected from the group consisting of carboxylic acids, amino acids, nucleic acids and mixtures of at least two of the afore-said acids.

9. Surgical thread according to any of the proceeding claims, **characterized in that** the self-sticking material further comprises a polymerization retarder, in particular selected from the group consisting of acids, acidic gases, acid anhydrides, polar substances, non-polar substances, ionic substances, non-ionic substances and mixtures of at least two of the afore-said polymerization retarders.

10. Surgical thread according to any of the claims 1 to 4, **characterized in that** the self-sticking material comprises a protein, in particular a cross-linkable protein, preferably gelatin and/or collagen.

11. Surgical thread according to claim 10, **characterized in that** the self-sticking material further comprises a crosslinking agent, in particular an enzyme, preferably a transglutaminase, being capable of crosslinking the protein, preferably cross-linkable protein, more preferably gelatin and/or collagen.

12. Surgical thread according to any of the preceding claims, **characterized in that** the self-sticking material is in the form of a hydrogel or in a dry, in particular lyophilized, form.

13. Surgical thread according to any of the preceding claims, **characterized in that** the surgical thread is in the form of a surgical suture.

14. Surgical device, in particular surgical suture, comprising at least one surgical thread according to any of the preceding claims.

15. Surgical kit comprising
- a surgical thread according to any of the claims 1 to 13 or a surgical device according to claim 14 and
- at least one further kit component selected from the group consisting of instructions for use, polymerization initiator, UV-Vis lamp, NIR lamp, instructions for use and combinations of at least two of the afore-said further kit components.
